# FASCICULE DE BREVET EUROPEEN

(11) **EP 2 210 610 B1**
(45) Date de publication et mention de la délivrance du brevet: **19.07.2017**
(21) Numéro de dépôt: 10305056.3
(22) Date de dépôt: 19.01.2010
(51) Int. Cl.: A61K 36/06, A61K 8/97, A61Q 19/06, A61P 17/00, A61K 8/60, A61K 8/73, A61K 8/02

(54) **UTILISATION D'UN EXTRAIT DE LENTINUS POUR LE TRAITEMENT DE LA CELLULITE ET DES AMAS GRAISSEUX .**
VERWENDUNG VON LENTINUSEXTRAKT ZUR BEHANDLUNG VON CELLULITIS UND FETTMASSE.
USE OF EXTRACT OF LENTINUS FOR THE TREATMENT OF CELLULITE AND FATTY MASSES.

(30) Priorité: 22.01.2009 FR 0950392
(43) Date de publication de la demande: 28.07.2010
(73) Titulaire: CASTER, 75008 Paris (FR)
(72) Inventeur: Choulot, Jean-Christophe, 78120, RAMBOUILLET (FR); Ales, Patrick, 75008, PARIS (FR)
(74) Mandataire: Dossmann, Gérard

(56) Documents cités:
- EP-A- 2 008 662
- EP-A- 2 017 290
- WO-A-2006/041526
- FR-A- 2 824 334
- FR-A- 2 908 771

## Description

La présente invention est relative à l'utilisation d'un extrait de Lentinus ainsi que la composition pharmaceutique qui le contient, dans le cadre de la lutte contre la cellulite et les amas graisseux sous-cutanés.

Les sirtuines sont des enzymes nucléaires appartenant à la famille des désacétylases dépendante du NAD+ que l'on trouve dans pratiquement tous les organismes, des bactéries jusqu'à l'Homme.

Ces protéines appelées « régulateurs silencieux d'information » (Silent Information Regulator), exercent leur activité enzymatique sur les histones mais également sur des régulateurs transcriptionnels, modulant par ce biais leur activité.

Les sirtuines sont en effet capables de déacétyler les histones sur lesquelles est enroulé l'ADN, le rendant moins sensible aux UV et aux ADNases.

Jusqu'à présent sept sirtuines, nommées SIRT1 à SIRT7, ont été identifiés chez l'Homme. Sur ces sept sirtuines, on s'intéresse surtout à la SIRT1, impliquée entre autres dans le vieillissement cutané (*Dal Farra C. et al., Sirt1, the human homologue to sirt2, is express in human skin and in cultured keratinocytes fibroblasts and HaCaT cells ; and its levels is closely related to stress and aging,* J. Cosmetic Scien., 2006, 57:187-188*).*

L'activité de désacétylase de SIRT-1 entraîne l'inhibition de la transcription des gènes impliqués ou « gene silencing ». Par ce mécanisme, SIRT-1 intervient dans la régulation de la protéine p53, facteur de transcription impliqué dans les voies cellulaires apoptotiques qui est acétylé et activé lors de stress cellulaires ou de dommages de l'ADN. SIRT-1 peut en effet induire la désacétylation de la protéine p53, c'est-à-dire à son inactivation, et bloquer sa transcription permettant ainsi de prolonger la longévité cellulaire (Cheng HL et al. Proc Natl Acad Sci USA. 2003).

L'ensemble des données bibliographiques indique clairement que SIRT1 est impliqué dans le processus du vieillissement (Porcu M. and Chiarugi A., Trends Pharmacol ScI, 2005; Langley E et al, EMBO J. 2002; HS Ghosh, Current Opinion in Investigational Drugs 2008 9:1095-1102; David Sidransky et al., Cell Cycle 5:17, 2005-2011, 1 September 2006)

L'expression élevée de SIRT1 dans les cellules jeunes contribue ainsi par une déacétylation de la protéine p53 et des histones à favoriser la longévité cellulaire. Cependant, l'expression de la protéine SIRT1 diminue au cours de vieillissement. Il a en effet été montré que des fibroblastes humains vieillis par passages réplicatifs successifs présentent une expression endogène réduite de la protéine SIRT-1 (Michishita et al., Mol Biol Cell. 2005 Oct;16(10):4623-35).

Chez les centenaires, la surexpression de certains gènes (comme IL10) ou de protéines particulières comme les sirtuines pourrait expliquer leur longévité.

SIRT1 est considérée donc comme une protéine de longévité qui:
- favorise la survie cellulaire et participe au système naturel de défense de l'ADN en désacétylant les histones sur lesquelles est enroulé l'ADN
- inhibe l'apoptose en désacétylant la protéine Ku70 « facteur de réparation d'ADN », ce qui permet à cette protéine de séquestrer dans le cytoplasme et d'inactiver le facteur Bax, connu pour favoriser l'apoptose (Brunet A et al., Cell. 1999;96: 857-68 ; Jeong J et al., Mol Med. 2007;39: 8-13);
- désacétyle la protéine nucléaire p53 impliquée également dans l'induction de l'apoptose.

La protéine SIRT1 agit également au niveau de la mitochondrie en détoxifiant l'organisme des radicaux libres. En effet, il a récemment été mis en évidence une voie de signalisation impliquant le cofacteur PGC-1, protéine clé de la biogenèse et du fonctionnement mitochondrial, et SIRT1 dont l'activation entraîne une amélioration du fonctionnement de la mitochondrie (Johan Auwerx et al., MEDECINE/SCIENCES 2007;23; 840-4).

La protéine SIRT1, possède également la capacité d'intervenir, au niveau de la cellule, dans le mécanisme qui contrôle l'accumulation des réserves adipeuses.

La restriction calorique augmente d'une part l'expression de SIRT1 dans une large gamme de tissus et cela modifie l'équilibre en faveur de la survie cellulaire (David A. Sinclair et al., Science 16 July 2004:Vol. 305. no. 5682, pp. 390 - 392). D'autre part, quand la cellule est privée de nourriture, SIRT1 diminue l'activité d'une autre molécule, le récepteur nucléaire PPAR-gamma, qui joue un rôle dans l'accumulation des graisses (Picard F et al. Nature 2004; 429:771-76).

On sait maintenant que SIRT 1 est identifiée comme médiateur de l'adipogénèse par son action sur la lipolyse par inhibition de la phosphodiesterase-4. Ainsi, au niveau adipocytaire, SIRT 1 inhibe l'adipogenèse et accroît la lipolyse (Qiao L. et al. J. Mol. Chem. 2006 Dec 29; 281 (52): 39915-24,). Comme les concentrations de SIRT1 diminuent au cours de la vie chez les Mammifères, ceci pourrait expliquer pourquoi nous prenons du poids avec l'âge.

La peau est l'organe de revêtement recouvrant la totalité de la surface du corps, constituée de trois compartiments distincts superposés : l'épiderme (épithélium de revêtement), le derme (tissu conjonctif de soutien) et l'hypoderme. L'épiderme est un épithélium stratifié qui constitue la structure externe de la peau et assure sa fonction de protection. Le derme est un tissu de soutien qui participe à la résistance, à l'élasticité et surtout à la tonicité de la peau et/ou des muqueuses. Au-dessous du derme se trouve une couche de tissus adipeux : l'hypoderme.

L'hypoderme est constitué d'une couche de tissu adipeux blanc organisé en lobules rattachés à la partie inférieure du derme par des travées de fibres de collagène et de fibres élastiques. Il est constitué des grosses cellules vacuolisées, les adipocytes, presque entièrement remplies de triglycérides. Ces cellules peuvent changer rapidement de volume, lors d'un amaigrissement ou d'une prise de poids, et peuvent mesurer de 40 à 120 µm de diamètre, ce qui correspond à une variation de 27 fois en volume.

Le tissu adipeux hypodermique constitue le plus grand réservoir énergétique de l'organisme. Il est capable de stocker des lipides sous forme de triglycérides par un processus de lipogénèse puis de les libérer sous forme d'acides gras et de glycérol par un processus de lipolyse. C'est l'équilibre entre ces deux voies métaboliques qui conditionne l'adiposité. Les réserves lipidiques de l'organisme se renouvellent en permanence et sont en relation étroites avec les apports nutritionnels et les besoins énergétiques de l'organisme.

Si un déséquilibre s'installe dans l'organisme entre les processus de lipogénèse et de lipolyse, le volume et le nombre des adipocytes peut augmenter; on parle d'hypertrophie et d'hyperplasie adipocytaire. Le développement excessif de la masse adipeuse peut alors se traduire par des modifications de l'aspect de la peau, voire évoluer vers une surcharge pondérale de l'individu pouvant aller jusqu'à l'obésité.

La cellulite, considérée comme inesthétique, correspond à une hypertrophie des cellules adipeuses par surcharge en triglycérides et une hyperviscosité de la substance fondamentale (par polymérisation des polysaccharides). Ces modifications gênent les échanges cellulaires et la mobilité des fibres conjonctives (collagène, élastine et réticuline), ce qui se traduit par une rétention d'eau, un ralentissement de la circulation veineuse et lymphatique et une perte de souplesse de la peau. L'accentuation du tissu adipeux est localisée, en particulier chez la femme, au niveau des hanches, des cuisses, des fesses, des genoux et des avant-bras. La peau prend un aspect matelassé et capitonné et au stade le plus avancé l'aspect de « peau d'orange », qui se caractérise par une succession de dépressions provoquées par un étirement excessif des travées conjonctives et l'attraction de l'épiderme vers les tissus profonds.

De nos jours, de nombreux efforts de recherche sont réalisés afin de trouver une manière efficace de lutter contre la cellulite et les surcharges graisseuses en général. De nombreuses méthodes ont été employées, comme par exemple certaines techniques médicochirurgicales telle que la lipoplastie, le drainage lymphatique, la mésothérapie, les techniques d'ionophorèse... Cependant ces techniques, bien qu'efficaces, sont lourdes et contraignantes.

Il subsiste un besoin tendant à agir d'une manière douce et non invasive, sur les mécanismes de formation des surcharges graisseuses sous-cutanées et de la cellulite.

Il a été envisagé d'agir sur le métabolisme des acides gras par des actifs cosmétiques et pharmaceutiques de façon à prévenir l'apparition de cellulite, par exemple en favorisant la lipolyse ou bien en inhibant la lipogenèse, c'est-à-dire en diminuant la formation des triglycérides.

Il subsiste cependant un besoin de composés permettant d'agir au niveau même de la formation et du stockage des surcharges graisseuses sous-cutanées.

La demanderesse a découvert que des extraits de Lentinus permettent une activation de l'expression de la protéine SIRT1 dans des cellules de kératinocytes. On envisage ainsi l'utilisation des activateurs d'expression de la protéine SIRT1 comme moyen de prévention ou de traitement local des dysfonctionnements métaboliques associés à la présence de cellulite et d'amas graisseux sous-cutanés.

La demanderesse a ainsi mis au point un extrait polysaccharidique issu de champignon du genre Lentinus pour son utilisation dans le traitement de la cellulite et des amas graisseux sous-cutanés.

L'invention a également pour objet l'utilisation d'une composition pharmaceutique contenant cet extrait.

D'autres objets apparaîtront à la lecture de la description, des dessins et des exemples qui suivent.

Les figures 1 et 2 montrent une analyse par spectrométrie de masse (MALDI-TOF) de l'extrait polysaccharidique obtenu dans l'exemple 1. Les poids moléculaires des composés visualisés s'étagent de 500 à 10 000 m/z (masse/charge).

Les figures 3 et 4 montrent des analyses chromatographiques de l'extrait polysaccharidique obtenu dans l'exemple 1. L'analyse résumée sur la figure 3 a été réalisée sur chromatographie liquide échangeuse d'ions. Celle de la figure 4 est une chromatographie d'exclusion stérique.

Les figures 5a et 5b montrent à différents grossissements (Fig5a : grossissement x20 ou Fig5a: grossissement x100) des analyses de l'expression de la protéine SIRT1 dans des kératinocytes en présence ou non d'un extrait de Lentinus selon l'invention.

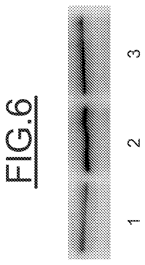

La figure 6 est une analyse semi-quantitative par une technique de western blot de la quantité de protéine SIRT1 présente dans les kératinocytes traitées ou non avec l'extrait polysaccharidique selon l'invention. La piste 1 montre la quantité de protéine SIRT-1 dans un témoin négatif, la piste 2, la quantité de SIRT-1 dans un témoin positif (révesratrol) et la piste 3 montre la quantité de la protéine SIRT-1 dans des kératinocytes traités par l'extrait polysaccharidique de Lentinus selon l'invention.

Lentinus edodes (Shii-take), un champignon forestier comestible du Japon, est connu dans de nombreux pays asiatiques (Chine, Corée, etc.) comme un des meilleurs champignons pour son goût et son parfum. Le Lentinus edodes est en effet caractérisé par quelques substances spécifiques, comme, par exemple, la guanosine 5'-monophosphate qui induit un parfum agréable et une substance aromatique, la lenthionine.

Récemment, les propriétés médicales du Shii-take ont été étudiées par des chercheurs japonais et on a pu mettre en évidence des actions antivirale, antibiotique, antitumorale et hypolipidémique.

Il est connu du brevet FR2776184 un extrait aqueux de Lentinus riche en lentinane, pour ses propriétés anti-tumorales. Le brevet FR2829389 décrit un procédé d'extraction de Lentinus comprenant des étapes d'hydrolyse enzymatique afin d'obtenir un extrait capable d'inhiber l'activité de certaines métalloprotéases, les MMPs et en particulier les MMP-1 et 2.

L'extrait polysaccharidique issu de champignon Lentinus, conforme à l'invention, est utilisé pour combattre la cellulite et les amas graisseux sous-cutanés d'un mammifère, et plus particulièrement de l'humain.

Cet extrait polysaccharidique, en activant l'expression de la protéine SIRT1 dans les cellules de peau, accroît à la fois l'inhibition de l'adipogenèse et la lipolyse.

L'extrait polysaccharidique issu de champignon du genre Lentinus, conforme à l'invention, comprend des monosaccharides, des polysaccharides, et en poids, de 2 à 8% de tréhalose, de 1 à 4 % de glucose et de 30 à 60 % d'alditols par rapport au poids total de l'extrait sec.

De préférence, cet extrait comprend, outre des monosaccharides et des polysaccharides, de 2 à 6 % de tréhalose, de 2 à 3 % de glucose et de 45 à 55 % d'alditols en poids par rapport au poids total de l'extrait sec. Plus préférentiellement, cet extrait comprend outre des monosaccharides et des polysaccharides, de 3 à 5 % de tréhalose, de 2 à 3 % de glucose et de 45 à 50 % d'alditols en poids par rapport au poids total de l'extrait sec.

De préférence, les alditols contenus dans cet extrait selon l'invention comprennent de l'arabinitol et du mannitol.

De préférence, l'extrait polysaccharidique selon l'invention est issu du champignon *Lentinus edodes.*

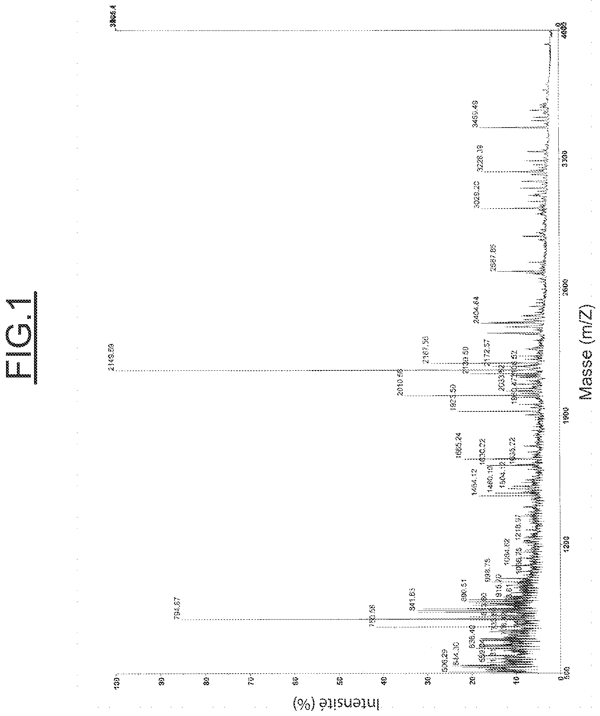

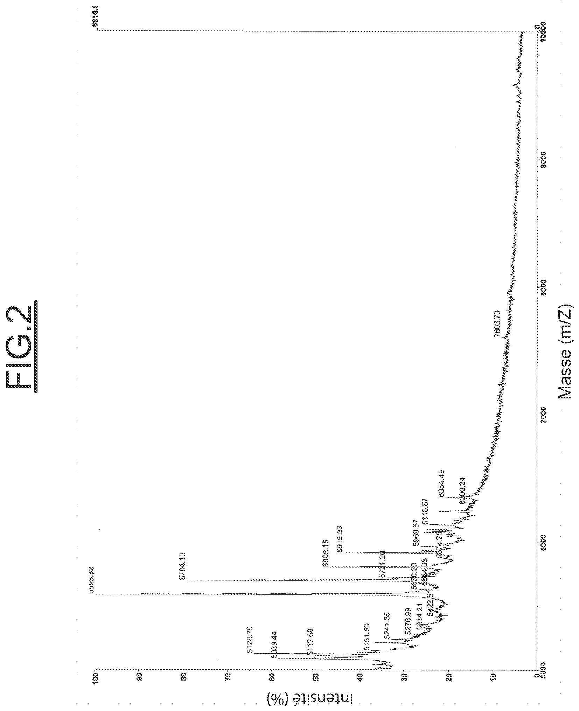

Un extrait polysaccharidique selon l'invention, particulièrement préféré, est défini par les analyses de spectrométrie de masse des figures 1 et 2.

L'extrait selon l'invention peut se présenter sous forme de poudre.

Le procédé d'extraction de l'extrait selon l'invention est décrit dans la demande de brevet n° FR 2 918 988.

Brièvement, on procède à une ou plusieurs extractions solide/liquide, l'extrait liquide est précipité par un solvant organique, centrifugé, filtré, puis le précipité est ensuite séché pour obtenir l'extrait polysaccharidique.

Dans un mode de réalisation de ce procédé, la ou les extractions s'effectuent à l'eau bouillante. De préférence, le solvant organique utilisé est de l'éthanol.

Les extraits sont obtenus à partir du procédé précédemment décrit à partir de champignons du genre Lentinus qui peuvent être utilisés séchés. De façon générale, les champignons frais contiennent 85 à 95% d'eau et les champignons séchés à l'air, entre 5 et 20% d'eau. De préférence, les champignons sont des Lentinus Edodes autrement nommés lentinula edodes, Bark, lentin du chêne, Shii-take, Shiitake, Cortinellus Shii-take ou Cortinellus edodes.

L'extrait polysaccharidique selon l'invention peut être utilisé dans un cadre cosmétique non thérapeutique pour combattre la cellulite et les amas graisseux sous-cutanés afin d'améliorer l'aspect esthétique du corps.

L'invention a également pour objet des compositions pharmaceutiques comprenant un tel extrait polysaccharidique, et éventuellement un véhicule inerte physiologiquement acceptable, pour leur utilisation dans le traitement d'humains ou de mammifères souffrant de cellulite ou d'un excès d'amas graisseux sous-cutanés.

L'extrait polysaccharidique selon l'invention peut être incorporé ou formulé dans un véhicule polymérique ou un système de délivrance pour une utilisation topique ou locale.

De telles compositions peuvent être utilisées à titre à titre préventif ou curatif.

Les compositions selon l'invention sont appliquées sur la peau ou surface cutanée d'un individu. Selon le mode d'administration, les compositions selon l'invention peuvent se présenter sous toutes les formes habituellement utilisées en cosmétique ou comme médicament. Ces compositions peuvent en particulier être formulées sous la forme de pommades, de crèmes, de laits, de gels.

Les exemples suivants illustrent l'invention sans la limiter aucunement.

### Exemple 1: Extraits totaux aqueux et précipité alcoolique

1 kilogramme de Lentinus edodes séché est broyé dans un broyeur à couteaux Henri. La poudre obtenue est extraite par 3 x 10 litres d'eau distillée bouillante. On agite 30 minutes à 90°C et on filtre sur un filtre Büchner de 50 litres sur une couche d'adjuvant de filtration (célite). On concentre à 5 litres dans un évaporateur à couche mince Luwa, puis on lyophilise dans un appareil à lyophiliser Serail. Une partie aliquote de chaque extrait aqueux est dissoute dans de l'eau distillée et précipitée par addition de 1,5 volume d'éthanol 96°. On laisse une nuit en chambre froide et on centrifuge (IEC Centra 7R). On sèche en dessiccateur sous vide.

### Exemple 2: Analyse de l'extrait selon l'invention par spectométrie de masse

L'extrait de l'exemple 1 a été analysé par spectrométrie de masse (MALDI-ToF). Le MALDI (Matrix Assisted Laser Desorption Ionization) est une méthode d'ionisation permettant d'analyser des molécules dont le poids moléculaire est supérieur à 1000 Da. L'analyseur couplé à la source MALDI est un analyseur à temps de vol (ToF, Time of Flight) qui est bien adapté à l'ionisation pulsée par désorption laser. La séparation dans un analyseur à temps de vol repose sur le fait que des ions de masse différente, accélérés à une énergie cinétique uniforme, ont des vitesses différentes, et donc un temps de vol différent pour parcourir une distance donnée.

Les figures 1 et 2 montrent une visualisation de l'ensemble des composées dont les poids moléculaires se situent entre 500 et 10000 m/z.

### Exemple 3: Caractérisation de l'extrait de l'exemple 1 par chromatographie

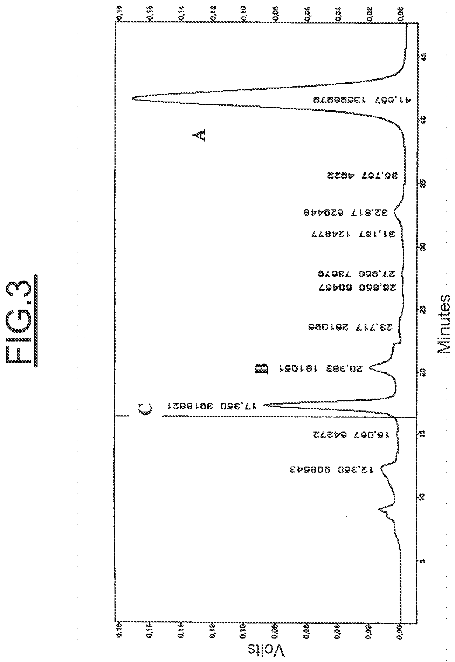

L'analyse de l'extrait brut de l'exemple 1 a été réalisée par la chromatographie liquide d'échange d'ions sur une colonne CARBO-Sep (CHO-682, Interchim) dont la sélectivité unique permet la séparation de mono- et disaccharides en utilisant seulement l'eau pure comme éluant. Le chromatogramme obtenu (Fig. 3) montre la présence majoritaire d'une petite molécule hydroxylée (A) et, entre autres, deux composés pouvant correspondre à un mono- (B) et un disaccharide (C).

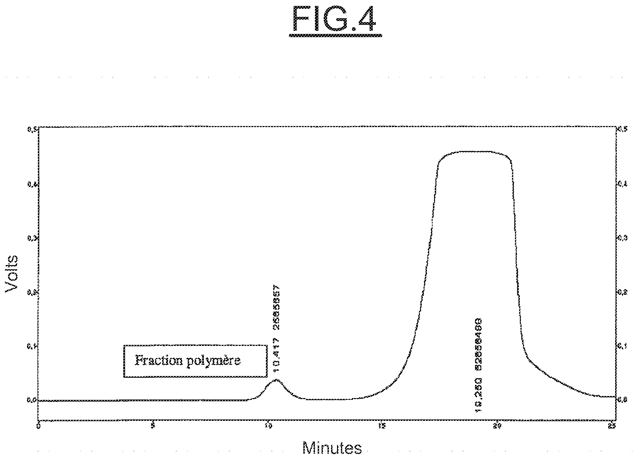

Dans un deuxième temps, l'analyse de l'extrait brut a été réalisée à l'aide de la chromatographie d'exclusion stérique sur la colonne Shodex OHpak B-804 (limite d'exclusion : 4 x 10⁵ kDalton). Le profil chromatographique obtenu montre que seulement 2% de l'extrait correspond à des polysaccharides (Fig. 4).

### Exemple 4: Ultrafiltrations de l'extrait de l'exemple 1

Les ultrafiltrations successives de l'extrait brut réalisées sur les membranes d'ultrafiltration en cellulose (Diaflo®-Amicon Co) caractérisées par trois seuils de coupure de 100 kDalton (kD), 10 kD et 1 kD ont conduit à l'obtention de 4 fractions (Lent. 100 = PM > 100 kD; Lent 10 = 10 kD < PM < 100 kD; Lent 1 = 1 kD < PM < 10 kD et Lent. UF = PM < 1 kD) dont la teneur en poids est présentée ci-dessous.

Pour chaque étape, le lavage à l'eau distillé est considérée comme terminé lorsque 10 volumes de liquide ont traversé la membrane.

**Tableau 1**

| Fractions | Lent. 100 | Lent. 10 | Lent. 1 | Lent. UF |
|---|---|---|---|---|
| Poids (mg) | 47 | 127 | 24 | 1874* |
| % | 2,4 | 6,5 | 1,2 | 89,9 |

| | | | | |
|---|---|---|---|---|
| * Après correction de la teneur en eau | | | | |

Les fractions contenant des polysaccharides, Lent.100 et Lent.10 correspondent à 8.9% (2.4% + 6.5%) du poids total de l'extrait actif.

Une hydrolyse acide totale de 4 ultrafiltrats suivie d'une analyse des sucres par chromatographie en phase liquide a été employée pour déterminer la nature des composantes glucidiques. La fraction Lent. UF, le plus important composant de l'extrait (89.9%), contient à part des monosaccharides (glucose, fucose, arabinose, mannose, xylose, arabinitol, mannitol) et disaccharides (tréhalose), des acides aminés, vitamines et des minéraux,

La composition des fractions Lent.1, Lent.10 et Lent.100 en sucres est présentée dans le Tableau 2.

**Tableau 2. Composition des fractions en sucres neutres**

| Sucres neutres | Rhamnose | Fucose | Ribose | Arabinose | Xylose | Mannose | Galactose | Glucose |
|---|---|---|---|---|---|---|---|---|
| Lent 1 | 12,3 | 10,4 | 5,2 | 2 | 2,6 | 20 | 32,5 | 15 |
| Lent 10 | 12,6 | 5,3 | 15,4 | 1,3 | 4,5 | 21 | 13,8 | 26,1 |
| Lent 100 | 4 | 5,1 | 7,1 | 5,4 | 4,6 | 17,1 | 26,4 | 29,3 |

| | | | | | | | | |
|---|---|---|---|---|---|---|---|---|
| * calculé sur la totalité des sucres neutres identifiés | | | | | | | | |

L'ensemble des résultats obtenus suite aux différentes analyses de l'extrait actif de l'exemple 1 est présenté dans le Tableau 3.

**Tableau 3**

| Constituants | (Lent. 100 + Lent 10) dont polysaccharides | Fraction > 1KD | Autres constituants | Tréhalose | Glucose | Autres monosaccharides | Alditols |
|---|---|---|---|---|---|---|---|
| En % | 8,9 | 1,2 | 34,1 | 4 | 2,6 | 2,9 | 46,3 |

### Exemple 5 : Evaluation par immunocytochimie de l'expression de SIRT-1 dans les kératinocytes humains

Des kératinocytes humains en lignée (HaCaT, German Cancer Research Center, Heidelberg, Germany) sont cultivés sur des lames de Lab-Tec (VWR International, Strasbourg, France) dans du milieu DMEM (Lonza, Saint Beauzire, France) supplémenté avec 10% de sérum foetal bovin (Lonza, Saint Beauzire, France) à 37°C dans une atmosphère humidifiée contenant 5 % de CO2.

Les kératinocytes (8000 cellules par puits) sont incubés avec l'extrait de Lentinus de l'exemple 1 à la concentration finale de 0.1% dans du milieu de culture pendant 24 heures, des cellules non traitées étant utilisées comme contrôles.

On procède à un lavage avec du PBS (phosphate-buffered saline) puis on fixe les kératinocytes avec du paraformaldéhyde (PFA) à 4%. Ensuite, on perméabilise les kératinocytes avec du Triton 0.1% (Sigma, Saint Quentin Fallavier, France).

On procède ensuite à l'immunomarquage en utilisant comme anticorps primaire un anticorps de lapin anti-SIRT1 humaine (dilution 1:50) (Santa Cruz Biotechnology, California, USA) et comme anticorps secondaire, un anticorps anti-lapin couplé à Alexa Fluor® 488 (dilution 1:50) (Invitrogen, Eragny-sur-Oise, France).

Les photographies ont été prises à l'aide d'un microscope à fluorescence (Nikon TE2000E) couplé à un appareil numérique (Nikon DXM1200F).

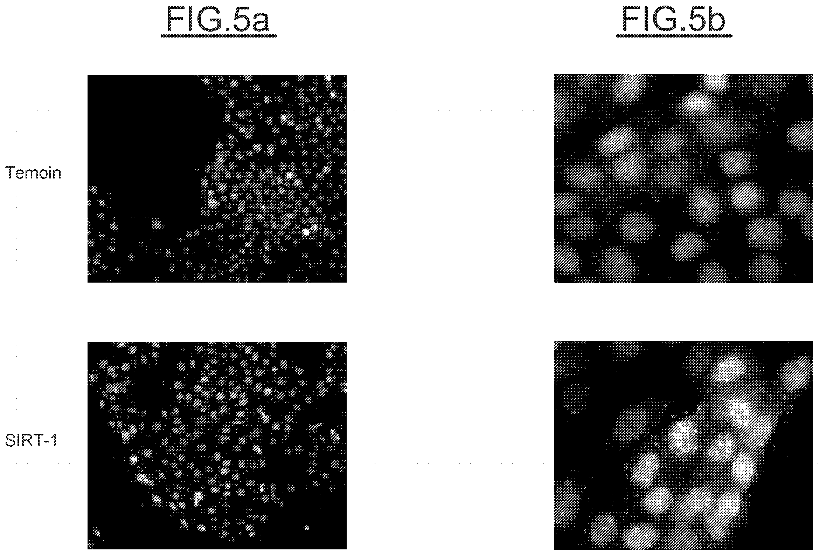

La figure 5 montre clairement aux différents grossissements que l'extrait de Lentinus selon l'invention provoque une expression de SIRT1 dans les cellules de kératinocytes par rapport au témoin.

### Exemple 6 : Analyse semi-quantitative de la protéine SIRT1 par la technique de Western blot :

Les Kératinocytes humains HaCaT) (10⁶ cellules) maintenus en culture dans un milieu DMEM (Lonza, Saint Beauzire, France) supplémenté avec 10 % de sérum foetal bovin (Lonza, Saint Beauzire, France) à 37°C dans une atmosphère humidifiée contenant 5 % de CO2, sont incubés comme précédemment avec l'extrait de Lentinus à la concentration finale de 0.1% dans du milieu de culture pendant 24 heures, des cellules identiques non traitées étant utilisées comme contrôles.

On procède ensuite à une lyse cellulaire avec 400 microlitres de tampon de lyse (Cell Lysis Buffer, Cell Signaling Technology, USA) additionné de 1mM de PhénylMéthylSulfonylFluorure (ou PMSF) qui est un inhibiteur irréversible de protéases (Sigma, Saint Quentin Fallavier, France).

Les protéines ainsi extraites sont centrifugées à 4°C à 10000 rpm durant 10 minutes et les surnageants obtenus sont stockés à -80°C avant utilisation.

Les protéines présentes dans 40 µg du lysat cellulaire (surnageant) sont séparées par l'électrophorèse sur gel de polyacrylamide-SDS (12%) et transférées ensuite sur une membrane PVDF « PolyVinyliDene Fluoride » (0.45 µm) Immobilon-FL, Millipore).

Les sites non spécifiques sont bloqués par incubation de la membrane avec du lait délipidé (Régilait, France) à 5% dans du TBST (Tris-Buffered Saline Tween-20) pendant la nuit à 4°C.

On incube ensuite la membrane avec un anticorps primaire de lapin anti-SIRT1 humaine (1/200) (Santa Cruz Biotechnology, California, USA) et puis avec un anticorps secondaire anti-lapin (dilution 1:50000) conjugué à la peroxydase HRP (Dako, Trappes, France).

La membrane est ensuite incubée avec un réactif de chimioluminescence. (Immobilon Western HRP Susbtrate, Millipore), puis la révélation et quantification de la bande de SIRT1 sont effectuées sur film autoradiographique à l'aide du système Molecular Imager ChemiDoc XRS (Bio-rad, France).

| | Name | Adj.Vol INT* mm² |
|---|---|---|
| Piste 1 | Contrôle négatif | 549.08 |
| Piste 2 | Contrôle positif | 2477.04 |
| Piste 3 | Extrait de Lentinus selon l'invention | 2303.44 |

Ces résultats montrent clairement une augmentation de 500% de la quantité de SIRT1 présente dans les kératinocytes traités avec l'extrait de Lentinus de l'exemple 1 (Piste 3) par rapport à celle quantifiée dans les cellules non-traitées (Piste 1).

### Exemple 7 Formulation d'une crème huile dans eau (exprimée en %)

| | |
|---|---|
| Eau | QSP 100 |
| Squalane | 5.00 |
| Petrolatum | 5.00 |
| Glycérine | 5.00 |
| Isodecyl Neopentanoate | 5.00 |
| Pentaerythrityl Tetraethylhexanoate | 5.00 |
| Cyclomethicone | 4.00 |
| Cetearyl Alcohol | 3.00 |
| Myristyl Myristate | 2.00 |
| Laureth-23 | 2.00 |
| Silica | 2.00 |
| Heptadecadienyl Furane | 0.1 à 10 |
| Cire d'abeille | 1.00 |
| Sclerotium Gomme | 1.00 |
| PEG-6 | 1.00 |
| Polyacrylamide | 0.80 |
| Glyceryl Stearate | 0.70 |
| Dimethiconol | 0.70 |
| Cetearyl Glucoside | 0.60 |
| C13-14 Isoparaffine | 0,40 |
| Acide Citrique | 0,14 |
| Laureth-7 | 0,10 |
| 4,5,7-Trihydroxyisoflavone | 0,01 à 10 |
| Caféine | 0.1 à 10 |
| Extrait d'Enteromorpha Compressa | 0.01 à 5 |
| Extrait de Garcinia Cambogia | 0.01 à 10 |
| Extrait de Ginkgo Biloba | 0.01 à 10 |
| Lentinus edodes | 0,02 à 5 |
| Extrait de fleurs de Sophora Japonica | 0.01 à 20 |
| Protéine de soja hydrolysée | 0.01 à 10 |
| Preservative system | QS |
| Parfum | QS |

### Exemple 8 : Formulation d'une crème eau dans huile (exprimée en %)

| | |
|---|---|
| Eau | QSP 100 |
| Polyisobutène Hydrogéné | 7.00 |
| Isocetyl Stearate | 7.00 |
| Cyclométhicone | 4.80 |
| Glycérine | 4.00 |
| Huile Minérale | 3.00 |
| Oxyde de Zinc | 3.00 |
| Butylene Glycol | 2.00 |
| Isononyl Isononanoate | 2.00 |
| Cire d'abeille | 2.00 |
| Cetyl Dimethicone Copolyol | 1.70 |
| Polyglyceryl-4 Isostearate | 1.65 |
| Hexyl laurate | 1.65 |
| Disodium tartrate | 1.60 |
| Chlorure de Sodium | 1.00 |
| PEG-6 | 1.00 |
| 4,5,7-Trihydroxyisoflavone | 0,01 à 10 |
| Retinyl palmitate | 0.01 à 10 |
| Lentinus edodes | 0,02 à 5 |
| Extrait d'Enteromorpha Compressa | 0.01 à 5 |
| Extrait de fleurs de Sophora Japonica | 0.01 à 20 |
| Extrait de Centella Asiatica | 0.01 à 5 |
| Protéine de Soja Hydrolysée | 0.01 à 10 |
| Preservative system | QS |
| Parfum | QS |

### Exemple 9 : Formulation d'un stick (exprimée en %)

| | |
|---|---|
| Huile de Castor | QSP 100 |
| Oleyl Alcohol | 20,00 |
| Hydrogenated Palm Kernel Oil | 17,00 |
| Cire de Candelilla | 11,00 |
| Polyglyceryl-3 Beeswax | 10,00 |
| Huile Minérale | 9,57 |
| Heptadecadienyl Furane | 0.1 à 10 |
| Karité | 2,00 |
| 4,5,7-Trihydroxyisoflavone | 0,01 à 10 |
| Quaternium-18 Hectorite | 1,10 |
| Titanium Dioxide | 1,00 |
| Acétate de Tocophéryl | 0,50 |
| Carbonate de Propylène | 0,33 |
| Parfum | QS |
| Rétinol | 0.01 à 10 |
| Lentinus edodes | 0,02 à 5 |
| Extrait d'Enteromorpha Compressa | 0.01 à 5 |
| Extrait de fleurs de Sophora Japonica | 0.01 à 20 |
| Extrait de Centella Asiatica | 0.01 à 5 |
| Protéine de Soja Hydrolysée | 0.01 à 10 |

### Exemple 10 : Formulation d'un gel crème (exprimée en %)

| | |
|---|---|
| Eau | QSP 100 |
| Cyclométhicone | 5,40 |
| Octyl Palmitate | 5,00 |
| Hydrogenated Coco-glycérides | 3,00 |
| Arachidyl Behenyl Alcohol | 2,55 |
| Propylène Glycol | 2,50 |
| Isodecyl Neopentanoate | 2,00 |
| Glyceryl Stearate | 1,70 |
| Cetyl Alcohol | 1,30 |
| Acide Stéarique | 1,00 |
| PEG-6 | 1.00 |
| Cire d'abeille | 0,40 |
| C13-14 Isoparaffine | 0,40 |
| Butylene Glycol | 0,16 |
| Glycérine | 0,16 |
| Cetearyl Alcohol | 0,10 |
| Cetyl Palmitate | 0,10 |
| Cocoglycerides | 0,10 |
| Laureth-7 | 0,10 |
| 4,5,7-Trihydroxyisoflavone | 0,01 à 10 |
| Extrait d'Enteromorpha Compressa | 0.01 à 5 |
| Lentinus edodes | 0,02 à 5 |
| Extrait de fleurs de Sophora Japonica | 0.01 à 20 |
| Extrait de Centella Asiatica | 0.01 à 5 |
| Protéine de Soja Hydrolysée | 0.01 à 10 |
| Preservative system | QS |
| Parfum | QS |

### Exemple 11 : Formulation d'un spray (exprimée en %)

| | |
|---|---|
| Eau | QSP 100 |
| Glycérine | 4.00 |
| Montmorillonite | 3.00 |
| PEG-6 | 3.00 |
| Glycine | 0.30 |
| Acide Citrique | 0.09 |
| 4,5,7-Trihydroxyisoflavone | 0,01 à 10 |
| Extrait d'Enteromorpha Compressa | 0.01 à 5 |
| Extrait de fleurs de Sophora Japonica | 0.01 à 20 |
| Extrait de Centella Asiatica | 0.01 à 5 |
| Protéine de Soja Hydrolysée | 0.01 à 10 |
| Preservative system | QS |
| Parfum | QS |

## Revendications

1. Extrait polysaccharidique issu de champignon du genre Lentinus comprenant des monosaccharides, des polysaccharides, et en poids, de 2 à 8 % de tréhalose, de 1 à 4 % de glucose et de 30 à 60 % d'alditols par rapport au poids total de l'extrait sec pour son utilisation dans le traitement de la cellulite et des amas graisseux sous-cutanés.

2. Extrait polysaccharidique pour son utilisation selon la revendication 1, **caractérisé par le fait qu'**il comprend en poids, de 2 à 6 % de tréhalose, de 2 à 3 % de glucose et de 45 à 55 % d'alditols par rapport au poids total de l'extrait sec.

3. Extrait polysaccharidique pour son utilisation selon l'une quelconque des revendications 1 à 2, **caractérisé par le fait qu'**il comprend en poids de 3 à 5 % de tréhalose, de 2 à 3 % de glucose et de 45 à 50 % d'alditols par rapport au poids total de l'extrait sec.

4. Extrait polysaccharidique pour son utilisation selon l'une quelconque des revendications 1 à 3, **caractérisé par le fait que** les alditols comprennent de l'arabinitol et du mannitol.

5. Extrait pour son utilisation selon l'une quelconque des revendications 1 à 4, qui est issu de champignon *Lentinus edodes.*

6. Extrait polysaccharidique pour son utilisation selon l'une quelconque des revendications 1 à 5, défini par les analyses de spectrométries de masse des figures 1 et 2.

7. Composition pharmaceutique contenant un extrait polysaccharidique tel que défini dans l'une quelconque des revendications 1 à 6, pour son utilisation dans le traitement de la cellulite et des amas graisseux sous-cutanés.

8. Composition pour son utilisation selon la revendication 7, dans laquelle le traitement est topique.

9. Utilisation cosmétique non-thérapeutique de l'extrait polysaccharidique tel que défini dans l'une quelconque des revendications 1 à 6 pour combattre la cellulite et les amas graisseux sous-cutanés.

## Patentansprüche

1. Polysaccharidextrakt aus einem Pilz der Gattung Lentinus, umfassend Monosaccharide, Polysaccharide sowie 2 bis 8 Gew.-% Trehalose, 1 bis 4 Gew.-% Glucose und 30 bis 60 Gew.-% Aldite in Bezug auf das Gesamtgewicht des Trockenextrakts, zur Verwendung in der Behandlung von Cellulite und Fettablagerungen unter der Haut.

2. Polysaccharidextrakt zur Verwendung nach Anspruch 1, **dadurch gekennzeichnet, dass** er 2 bis 6 Gew.-% Trehalose, 2 bis 3 Gew.-% Glucose und 45 bis 55 Gew.-% Aldite in Bezug auf das Gesamtgewicht des Trockenextrakts umfasst.

3. Polysaccharidextrakt zur Verwendung nach einem der Ansprüche 1 bis 2, **dadurch gekennzeichnet, dass** er 3 bis 5 Gew.-% Trehalose, 2 bis 3 Gew.-% Glucose und 45 bis 50 Gew.-% Aldite in Bezug auf das Gesamtgewicht des Trockenextrakts umfasst.

4. Polysaccharidextrakt zur Verwendung nach einem der Ansprüche 1 bis 3, **dadurch gekennzeichnet, dass** die Aldite Arabinit und Mannit umfassen.

5. Extrakt zur Verwendung nach einem der Ansprüche 1 bis 4, der aus dem Pilz *Lentinus edodes* stammt.

6. Polysaccharidextrakt zur Verwendung nach einem der Ansprüche 1 bis 5, der durch die massenspektrometrischen Analysen der Figuren 1 und 2 definiert ist.

7. Pharmazeutische Zusammensetzung, enthaltend einen Polysaccharidextrakt wie in einem der Ansprüche 1 bis 6 definiert, zur Verwendung in der Behandlung von Cellulite und Fettablagerungen unter der Haut.

8. Zusammensetzung zur Verwendung nach Anspruch 7, wobei die Behandlung topisch erfolgt.

9. Nichttherapeutische kosmetische Verwendung des Polysaccharidextrakts wie in einem der Ansprüche 1 bis 6 definiert zur Bekämpfung von Cellulite und Fettablagerungen unter der Haut.

## Claims

1. Polysaccharide extract derived from fungus of the genus Lentinus comprising monosaccharides, polysaccharides and, by weight, from 2 to 8% of trehalose, from 1 to 4% of glucose and from 30 to 60% of alditols relative to the total weight of the dry extract for its use in the treatment of cellulite and subcutaneous accumulations of fat.

2. Polysaccharide extract for its use according to Claim 1, **characterized in that** it comprises, by weight, from 2 to 6% of trehalose, from 2 to 3% of glucose and from 45 to 55% of alditols relative to the total weight of the dry extract.

3. Polysaccharide extract for its use according to either of Claims 1 or 2, **characterized in that** it comprises, by weight, from 3 to 5% of trehalose, from 2 to 3% of glucose and from 45 to 50% of alditols relative to the total weight of the dry extract.

4. Polysaccharide extract for its use according to any one of Claims 1 to 3, **characterized in that** the alditols comprise arabinitol and mannitol.

5. Extract for its use according to any one of Claims 1 to 4, which is derived from the mushroom *Lentinus edodes.*

6. Polysaccharide extract for its use according to any one of Claims 1 to 5, defined by the mass spectrometry analyses in Figs. 1 and 2.

7. Pharmaceutical composition containing a polysaccharide extract as defined in any one of Claims 1 to 6, for its use in the treatment of cellulite and subcutaneous accumulations of fat.

8. Composition for its use according to Claim 7, in which the treatment is topical.

9. Non-therapeutic cosmetic use of the polysaccharide extract as defined in any one of Claims 1 to 6 for combating cellulite and subcutaneous accumulations of fat.
